# EUROPEAN PATENT SPECIFICATION

(11) **EP 1 124 529 B1**
(45) Date of publication and mention of the grant of the patent: **09.01.2008**
(21) Application number: 99957492.4
(22) Date of filing: 01.11.1999
(51) Int. Cl.: A61K 6/083, C01B 13/32, C08K 7/00

(54) **IMPROVED FILLER FOR DENTAL COMPOSITE MATERIALS**
FÜLLSTOFFE FÜR DENTALKOMPOSITEN
CHARGE AMELIOREE DESTINEE AUX MATERIAUX COMPOSITES DENTAIRES

(30) Priority: 03.11.1998 US 106806 P
(43) Date of publication of application: 22.08.2001
(73) Proprietor: New Age Biomaterials, Inc., Halifax, Nova Scotia B3H 2X1 (CA)
(72) Inventor: JONES, Derek, W., Halifax, Nova Scotia B3H 2X1 (CA); RIZKALLA, Amin, S., Halifax, Nova Scotia B3L 4T5 (CA); HALL, Gordon, C., Lower Sackville, Nova Scotia B4C 3G6 (CA)
(74) Representative: Hartz, Nikolai
(86) International application number: PCT/US1999/025601
(87) International publication number: WO 2000/025729

(56) References cited:
- WO-A-96/26237
- GB-A- 2 291 053
- US-A- 4 215 033

## Description

### FIELD OF INVENTION

The present invention relates to dental products and processes and more particularly, to the fabrication of ceramic particulate materials. More particularly, the invention relates to dental filling materials formed from a resin matrix and a ceramic or glass filler in the form of a composite restorative material. More particularly, the invention relates to the production of specific shapes of ceramic particulate material ranging in size from sub-micron to about 50µm, but preferably with a mean size of about 7µm.

### BACKGROUND OF THE INVENTION

GB-A 2 291 053 discloses an inorganic filler composition. WO 96/26237 discloses a composite composition containing shaped filler particles.

This invention deals with development of an improved resin glass or ceramic composite system which has wide application and may be used for dental restorative filling materials for teeth and biomedical bone cement. It will be appreciated by one skilled in the art that modern 'dental composite' materials are a blend of glass and/or ceramic particles dispersed in a polymerizable synthetic organic resin. The polymer materials are blended together with the finely divided inorganic material such as a barium aluminosilicate or zirconium silicate glass or other glass ceramic compositions having an effective amount of radiopacifying agent that renders the resultant glass radiopaque to X-rays. Such dental restorative composite materials comprised of a blend of liquid polymerizable organic binder and a solid inorganic filler are known to the prior art. Such compositions are described in general terms for example in U.S. Pat. No. 3,066,112 and US-A 4,215,033. The full potential application in dentistry of glass and glass/ceramic/resin composite biomaterials has not yet been achieved because the current composite materials cannot completely withstand the aggressive environment of the oral cavity. Major shortcomings are low fracture toughness and the inability of the composite materials to resist abrasion and wear in the mouth.

The dental restorative composite materials using the improved filler particles of this invention may be prepared according to known methods of the prior art such as employed in US Pat. No. 3, 066, 112. The improved composite restorative system having a tooth-like colour can be used to replace the conventional amalgam or gold dental fillings. Materials such as amalgam suffer from uncertainty as to the biological effect of the introduction of mercury into the oral cavity over long periods of time. In addition the metallic hue of amalgam restorations is not aesthetic.

Currently dental composite systems suffer from lack of sufficient adhesion being established between the inorganic (glass or ceramic) filler and the resin matrix. The modulus of elasticity of a composite material will show the effectiveness of the stress/strain transfer from matrix to the filler particles. The modulus of elasticity and Poisson's ratio of dental restorative materials are also regarded as important fundamental properties, because a material with a low elastic modulus will more readily elastically deform under a given masticatory functional force. Excessive elastic deformation of the restorative material under functional stress may result in catastrophic fracture of surrounding brittle tooth enamel structure, or alternatively increased microleakage may result. The increased use of polymer/glass composite systems as posterior restoratives (in back 'molar' grinding teeth) which are subjected to much higher levels of force than anterior restorations, might suggest the use of materials with a higher modulus of elasticity and fracture toughness in order to minimize the risk of cusp fractures. A dental restorative composite material with a higher modulus of elasticity and fracture toughness will be able to provide support at the interface with tooth enamel to protect the enamel rods at the margin from fracturing.

Excessive wear of the restoration due to loss of filler particles (pull-out) followed by easier wearing away of the softer resin matrix is another problem in such situations.

A major limitation of the current dental composite materials is the relatively low fracture toughness. Fracture toughness is the energy absorbed by the material in resisting crack propagation. Dental restorative composite materials exhibiting higher fracture toughness values will have a better resistance to fracture and functional wear.

### OBJECTS OF THE INVENTION

None of the composites heretofore known in the art disclose or suggest the novel method for producing the unique filler particles for composites as in the present invention. An object of this invention therefore, is to provide a ceramic filler for a composite resin material exhibiting a capability to mechanically lock into the resin matrix but at the same time not produce the high stress concentration around the filler that can occur with an irregular shaped filler. The incorporation of the unique shaped particles will significantly increase the fracture toughness and wear resistance of the composite.

These and other objects of the invention, which shall become apparent from the description to follow, are achieved by the invention as hereinafter described and claimed.

### SUMMARY OF THE INVENTION

The present invention provides a filled dental material as defined by the claims.

In general, the present invention is based on a method to synthesize ceramic filler components with a specific shape, such that it will allow mechanical interlocking into the organic resin matrix. The incorporation into a resin composite of from 5 to 35% by weight of these unique shaped particles together with conventional glass filler will result in a significantly higher fracture toughness and improved wear resistance. Specific shaped particles can also be incorporated to control consistency to produce a flowable or packable composite material. In addition, spherical particles of silica, alumina or zirconium, titanium, barium or strontium silicate synthesized by wet chemical methods can also be blended together with the specific shaped type of ceramic filler to improve packing density. The invention provide unique filler particle for use in dental composites.

### BRIEF DESCRIPTION OF THE ILLUSTRATIONS

Figure 1 is a SEM image example of doughnut-shaped silica ceramic particles according to the present invention. Mean size being about 5 µm with a few larger particles being about 15 µm.
Figure 2 is a SEM image example of doughnut-shaped zirconium silicate ceramic particles. Mean size being about 5 µm.
Figure 3 illustrates a SEM image example of alumina doughnut-shaped particles, which have mean size of about 5 µm.
Figure 4 illustrates a SEM image example of a mixture of alumina spherical and doughnut-shaped particles with a similar size and distribution.
Figure 5 and 6 illustrate SEM image examples of silica doughnut-shaped particles that are coated with zirconium silicate spicules. Mean size being about 5 µm with a few larger particles being about 15 µm.
Figure 7 to 12 illustrate SEM image examples of multi-dimpled shaped ceramic particles of zirconium silicate according to the present invention with mean particle size of about 6 to 7 µm.
Figure 13 illustrates SEM image examples of multi-dimpled and spherical shaped mullite ceramic particles. Mean particle size of about 2 µm.
Figure 14 illustrates a SEM image example of barium silicate porous multi-dimpled shaped particles ranging from 0.5 to 5 µm.
Figure 15 is a SEM illustrating examples of mono-sized silica spherical particles with a size of about 0.3 µm. (Reference)
Figure 16 is a SEM impage illustrating examples of zirconium silicate spherical particles (Reference).
Figures 17 and 18 illustrate SEM image examples of nugget shaped particles of strontium and barium silicates.
Figures 19 and 20 illustrate SEM image examples of zirconium silicate rod and fiber-shaped particles (Reference).
Figure 21 illustrates a SEM image example of hollow semi-spherical crystalline robust heavy plate-like shaped particles of barium silicate (Reference).
Figures 22 to 29 illustrate SEM image examples of barium silicate hollow spherical shaped particles with a delicate porous mesh surface.
Figure 30 illustrates a SEM image example of barium silicate hollow spherical porous "ball of wool-like" particles.

The above characteristic shapes can be consistently reproduced, the size and chemical composition can be varied by changing the conditions of synthesis.

The above shapes can be produced with various chemical compositions.

### PREFERRED EMBODIMENT FOR CARRYING OUT THE INVENTION

The present invention provides a method for producing discrete unique ceramic particles having specific size, shape, topography and chemistry. These particles can be used as the reinforcing phase in a resin matrix composite for use as a dental filling material and other non-dental applications. Some particles will require to possess the property of radiopacity, to allow diagnostic X-ray images to be produced of fillings in teeth to permit an assessment of the presence of any further dental decay adjacent to the filling. The ceramic particles are specifically designed to function as a reinforcing strengthening phase in a resin/ceramic composite material. These particles represent a unique and novel approach to the production of ceramic filler particles for dental composite biomaterials. The ceramic particles will possess unique shape and texture characteristics not previously in existence for dental composite filling materials. The ceramic particles will lead to the technological development of significantly improved dental composite materials with enhanced fracture toughness and wear resistance.

This invention concerns an aqueous acid solution or suspension of precursors for producing glass, glass-ceramic or ceramic particulate materials. The aqueous acid solution or suspension comprises a combination of all the precursors necessary to give a finished product when the precursor composition is dried and fired. This invention is not limited to any particular wet-chemical method of synthesis or any particular precursors for any specific glass, glass-ceramic or ceramic particulate materials. On the contrary, any combination of precursors or wet chemical synthesis methods can be employed which will result in unique shaped particles for use as a reinforcing phase in a ceramic or glass-ceramic or ceramic/resin composite.

At least six basic wet chemical synthesis methods may be used to produce ceramic or glass filler particles, the term "wet-chemical synthesis" means any of the following six methods: 1) sol-gel polymerization of prehydrolyzed alkoxides (some soluble metal salts may be added becoming complexed into the gel). 2) The precipitation of precursors from suspensions by spray-drying, spray-freeze drying or freeze-drying. 3) Room temperature or elevated aqueous solution precipitation synthesis methods. 4) Hydrothermal synthesis in which the aqueous solutions or suspensions of precursor materials are heated at elevated temperatures and pressures. 5) Organic solution synthesis precipitation methods, and 6) Glycothermal synthesis in which the organic solutions or suspensions of precursor materials are heated at elevated temperatures and pressures. These methods allow homogeneous glasses, ceramics and glass-ceramics to be formed at temperatures well below the normal temperature required to sinter high density bodies of uniform microstructure. The unique shapes are achieved by careful control of the parameters such as: chemistry of starting solution, the viscosity and age of the solution, the concentration of the solution, the temperature used during the dehydration process, and the like. The term "shaped particles" means that the particles possess a distinct shape and topography which lends itself to aiding the mechanical locking of the resin matrix with the particle. For the sake of brevity the terms glass or ceramic are employed to refer to any combination of inorganic glass, ceramic or glass-ceramic structure. The term "aqueous solution" means an aqueous solution, suspension or dispersion which may be acidic, neutral or basic, which may contain a range of salts and polymeric substances. This invention also concerns specific shaped particles formed during dehydration of the aqueous solution and or during subsequent calcining of the glass precursor powder. This invention also concerns shaped particles of glass or ceramic made by spray-drying or other wet chemical method of synthesis in which the precursor aqueous solution is subsequently dehydrated and calcined producing a precursor powder.

The aqueous solutions of this invention preferably have a pH typically below about 5. Preferred acids for pH control are those that decompose cleanly on heating and leave no residue that would require prolonged calcination.

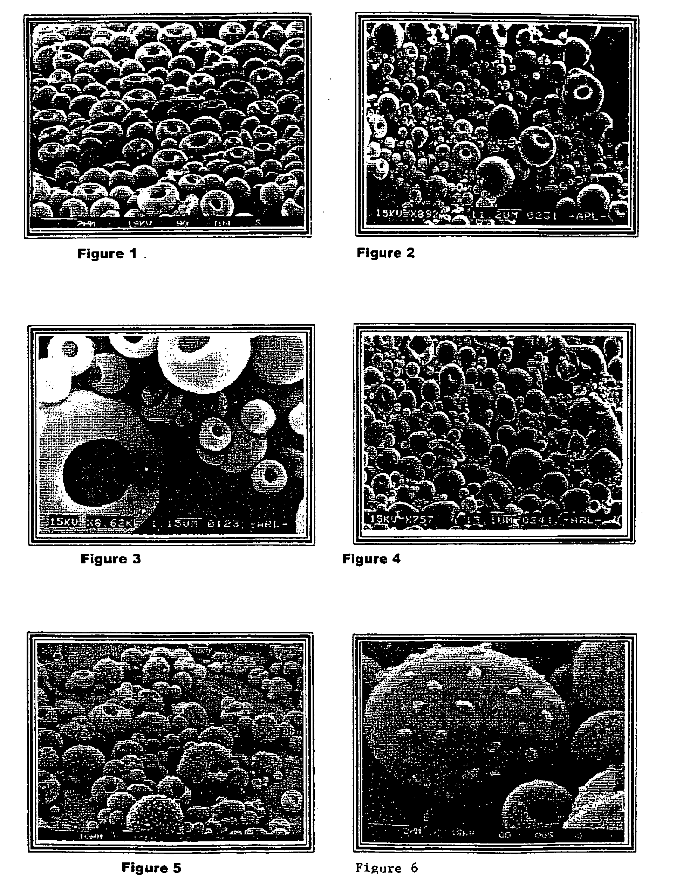

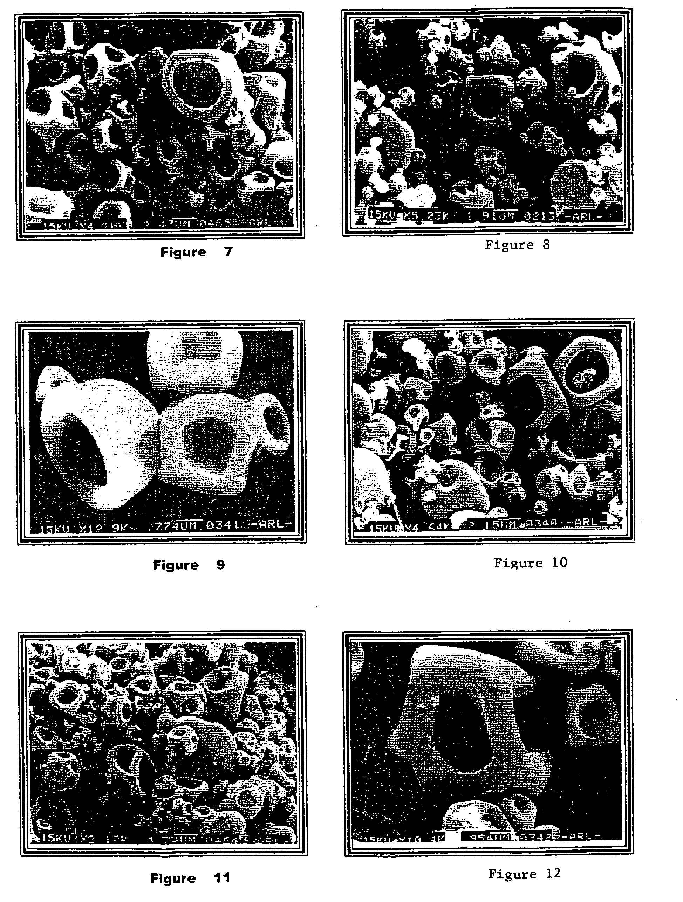

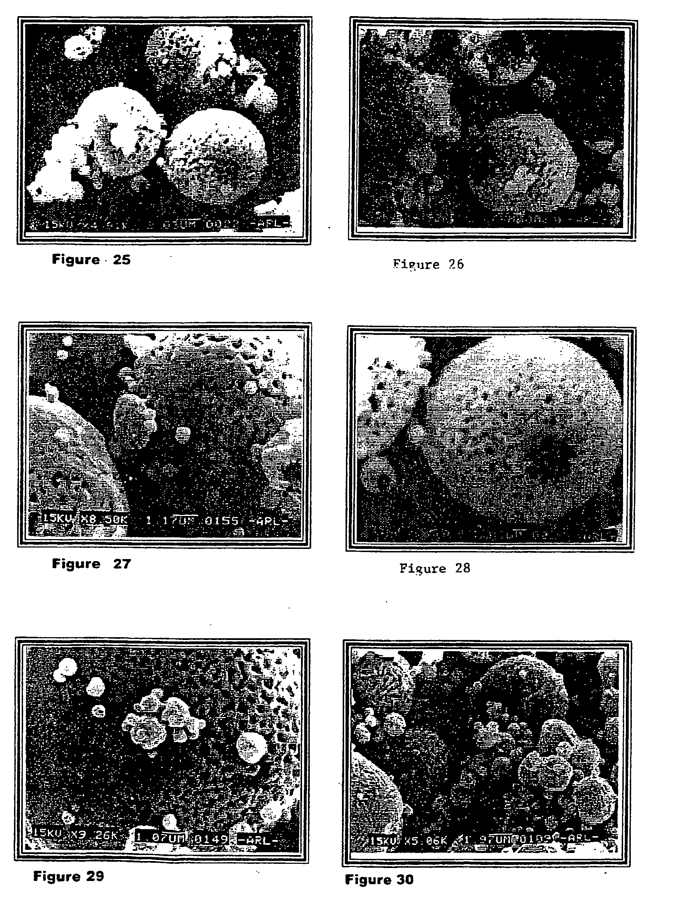

A variety of unique shaped filler particles are employed such as: doughnut, spherical, multi-dimpled, porous hollow spheres and nugget whereby doughnut, multi-dimpled porous hollow spheres or nuggets are present. The chemistry of these particles can be, alumina, mullite, silica, or silica compounds containing zirconium, strontium, barium, titanium, or combinations of similar elements and compounds. The appearance and size of the particles shown in the SEM images Figures 1 to 30 are for illustrative purposes only and are not intended to covey absolute limitations of the invention. In this regard, it is understood that each individual particle of a given shape can vary up to about 50 micrometers or larger. Preferred particles sizes will be provided below.

A major feature is the production of unique shaped ceramic particles which have a very reproducible controlled shape, particle size and distribution with no need for grinding or sieving. The silica, alumina, zirconium silicate, barium silicate, strontium silicate and mullite ceramic particles produced using wet chemistry possessing unique shapes for use as fillers in composite systems provide mechanical locking for the filler within the resin matrix. These customized shapes have the capability of preventing pull-out from the resin matrix during abrasion. Tests have been conducted which indicated that composite formulations containing unique shaped particles demonstrate excellent abrasion resistance and fracture toughness.

The following seven types of zirconium silicate particles have been produced with unique characteristic shapes.
1) Reference Spherical (25% Zr oxide)
2) Reference Spherical (15% Zr oxide)
3) Reference Spherical (8% Zr oxide)
4) Multi dimple (25% Zr oxide)
5) Multi dimple (15% Zr oxide)
6) Mixture of spherical and multi dimple (8% Zr oxide)
7) Mixture of spherical and multi dimple (4% Zr oxide)
8) Doughnut (4% Zr oxide)
9) Reference Rods or fibre (25% Zr oxide)
10) Reference Rods or fibre (35% Zr oxide)

Examples of typical mean particle size and the mean size of the 10 and 90 percentiles are shown in Table 1. The particle size and distribution is extremely reproducible and can also be varied within a limited range by changes in parameters of flow rate and concentration of solution or suspension.

**TABLE 1. Examples of typical mean particle size.**

| Particle Type | Mean µm | 10% Mean µm | 90% Mean µm |
|---|---|---|---|
| 1 spherical | 7.69 | 1.93 | 14.14 |
| 4 Multidimple | 7.77 | 2.08 | 14.14 |
| 5 Multidimple | 6.1 | 1.84 | 11.00 |
| 6 Mixture | 4.63 | 1.3 | 9.13 |
| 7 Mixture | 5.34 | 1.32 | 10.82 |
| 8 Doughnut | 5.14 | 1.80 | 8.81 |
| 9 Rods | Length 15-25 µm Diam. 0.3-4 µm | | |

The three major variables affecting the shape of particles are the time of the reaction or the age of the solution, the pH and the actual chemistry of the solution. The longer time or older solutions give the unique multi-dimple shapes, while the shorter times give spherical particles. The much longer time or much older solutions containing the higher percentage of zirconium produce particles which include the rod or fibres.

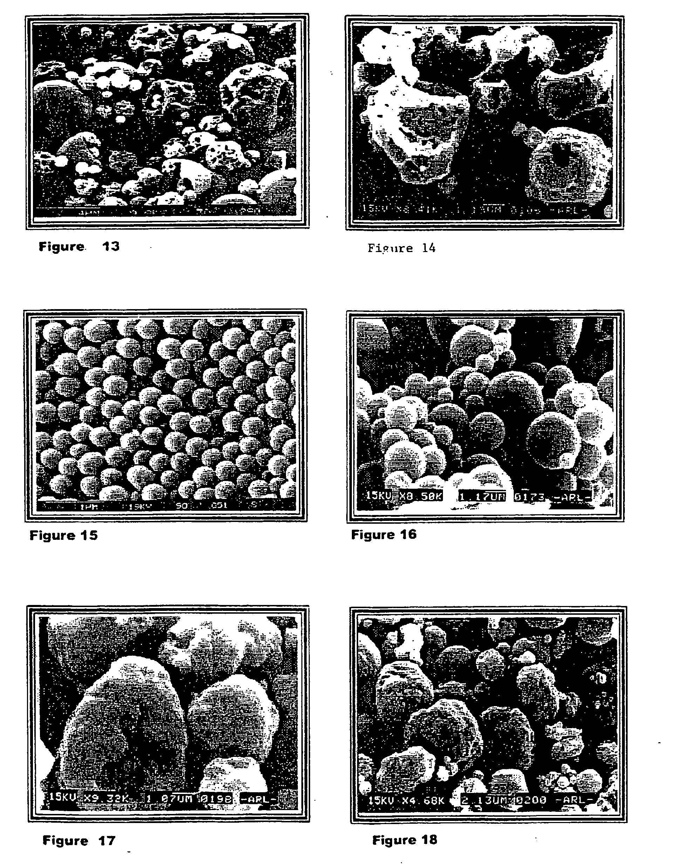

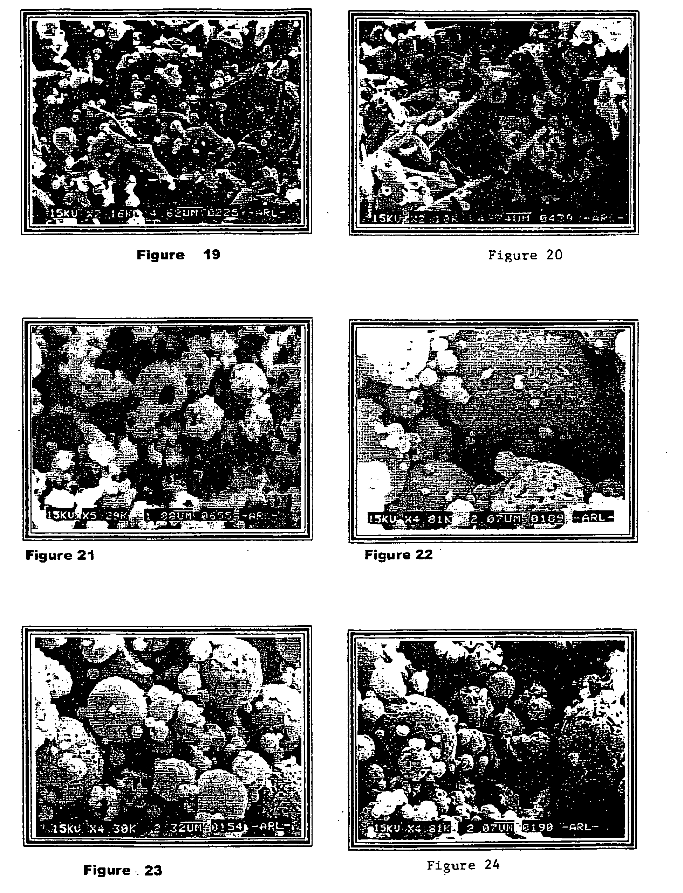

The barium silicate particles containing 70, 60, and 40% and 4% BaO and strontium silicate containing 4% SrO exhibit the following nine types of particle shapes.
1) 70% BaO - Hollow semi-spherical cyrstalline heavy robust plate-like structure, mean particle size 1-3 µm (Figure 21). (Reference)
2) 60% BaO - Hollow spherical delicate mesh structure, mean particle size 2-5 µm (figures 22 to 29).
3) 60% BaO - Hollow spherical delicate mesh structure combined with multi-dimple shapes, mean particle size 2-5 µm.
4) 40% BaO - Mixture of non-porous spherical multi-dimple and doughnut shapes, mean particle size 2-5 µm.
5) 40% BaO - Mixture of porous spherical multi-dimple and some doughnut shapes, mean particle size 3-6 µm.
6) 4% BaO non-porous spherical particles, typically 1 to 10 µm, with a mean size of about 4 µm. (Reference)
7) 4% BaO 'Nugget Shaped' particles typically 1 to 10 µm, with a mean size of about 4 µm (Figure 18).
8) 4% SrO non-porous spherical particles typically 1 to 10 µm, with a mean size of about 4 µm. (Reference)
9) 4% SrO 'Nugget Shaped' particles, typically 1 to 10 µm, with a mean size of about 4 µm (Figure 17).

The first of the barium silicate shapes is best described as a spherical mesh-like structure built up from what looks like crystalline plate-like structures. The second is a general spherical shape possessing a porous surface and a third exhibits a delicate mesh structure combined with some multi-dimple shapes. The fourth versions comprise a mixture of non-porous spherical particles together with doughnut, and multi-dimple shapes. The fifth version of the barium silicate (40% BaO) particles differs from the fourth type in that the spherical shapes exhibit porosity. Tests have confirmed the consistency and reproducibility of synthesizing the shape and texture for these particles. These barium silicate particles provide a structure for the polymerizable resin to penetrate and mechanically lock into. X-ray analysis has confirmed the crystalline nature of the first type of unique barium silicate particles. Adequate radio-opacity is considered to be mandatory for posterior dental composite materials in facilitating the diagnosis of secondary caries adjacent to the restoration. Translucency is not particularly important for posterior restorations, from the point of view of aesthetics; however, for curing systems it is important to achieve an adequate depth of cure. Thus, translucency has to be balanced with the need to have adequate radio-opacity.

Surface area: The multi-dimpled zirconium particles have a surface area in the range of 2-3 m²/g, which is greater than two of the spherical shaped (non-dimpled) particles of zirconium silicate. The combination of multi-dimple and rod like particles gave a significantly higher surface area of 18.06 m²/g. the different forms of the barium silicate particles have surface areas ranging from 3.10 to 4.37 m²g, which is generally greater than for the zirconium silicate particles, the exception being the combination of multi-dimple and rod like zirconium silicate particles.

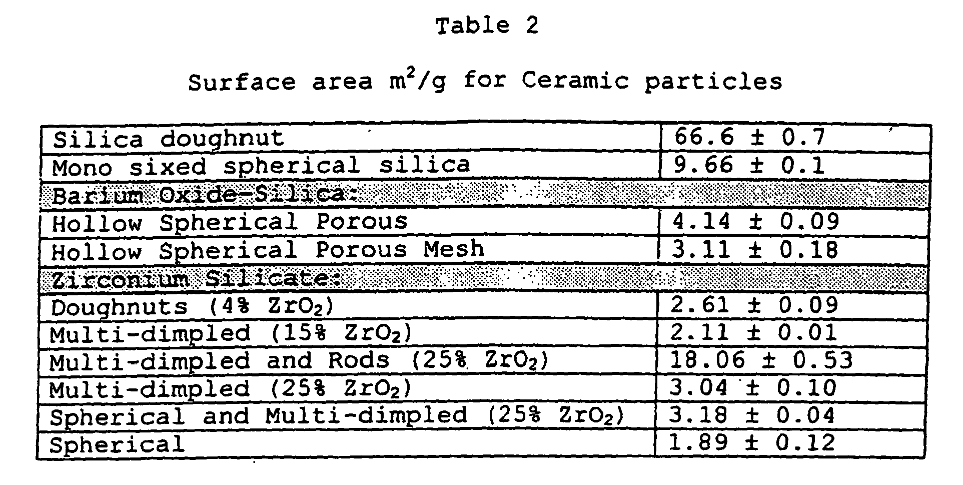

The examples of the various surface areas exhibited by the different forms of particles of this invention shown in Table 2 allow for the blending of different particles to optimize the desired consistency when mixed with the polymer resin.

Modulus of elasticity tests have confirmed that the customized silica, alumina, mullite, zirconium silicate, barium silicate strontium and titanium silicate ceramic particles produced by wet chemistry synthesis possessing unique shapes for use as a fillers in composite systems provide mechanical locking for the filler within the resin matrix.

These shapes have shown that they provide improved mechanical performance for a resin/glass or ceramic composite systems. The customized shapes should prevent pull-out from the resin matrix during abrasion.

Excessive elastic deformation of the restorative material under functional stress may result in catastrophic fracture of surrounding brittle tooth enamel structure, or alternatively increased microleakage may result. However, it is the property of fracture toughness which is the most important mechanical property. Currently dental composite an dental amalgam restorativematerials have fracture toughness K_{IC} values well below 2 MPa .m^{1/2}. The incorporation of 20% of the unique shaped ceramic barium silicate filler of this invention into a composite formulation has produced fracture toughness K_{IC} values of over 3 MPa.m^{1/2}. Fracture toughness values for 10 commercial dental composite materials together with an experimental formulation containing 20% of the unique shaped barium silicate hollow porous spheres are illustrated in Table 3 below.

**Table 3**

| Fracture Toughness of composite Materials | |
|---|---|
| Materials | Fracture Toughness (K_{1c}) MPa.m^{1/2} |
| Charisma F | 1.18 ± 0.35 |
| Herculite XR Unidose | 1.26 ± 0.38 |
| Herculite XRV | 1.66 ± 0.32 |
| P-50 | 0.76 ± 0.08 |
| Silux Plus | 0.85 ± 0.11 |
| Solitaire | 0.72 ± 0.26 |
| Surefil | 1.56 ± 0.23 |
| Tetric Ceram | 1.37 ± 0.36 |
| TPH | 1.89 ± 0.18 |
| Z-100 | 1.11 ± 0.17 |

Experimental composite containing 20% (wt) barium silicate 'Hollow Porous Mesh Spheres' gave K_{IC} values in excess of 3 MPa.m^{1/2}.

Current dental composites cannot withstand the aggressive environment of the oral cavity. Improved dental composite restoratives with significantly improved fracture toughness values and increased resistance to wear and abrasion are now possible due to the development of the unique shaped filler particles of this invention.

Specifically 5-35% of the unique shaped filler paticles of this invention blened with conventional ground glass filler particles can provide vastly improved fracture toughness, excellent radio opacity (of at least 3 mm equivalent aluminium), as well as significantly imrpoving the resistance to wear.

Some dental composites are marketed as possessing a high packability force which simulates the handling characteristics of dental amalgam during placement. Other dental composite materials are marketed as flowable materials. A consistency test which evaluates the packability force has been used to compare five commerical composte materials with the experimental shaped particles (62 and 68% wt filler loading). As illustrated in the table below the Surefil (DENTSPLY) material exhibited the hightest value for any of the commercial materials but was only one third of the value for the experimental doughnut shaped material. The consistency can be influenced by the volume, shaped-size (surface area), and size distribution of the filler particles incorporated. The two experimental shaped ceramic particles, doughnut (109 N) of this invention and mono-sized spheres (4.6 N) can be used to blend with other ceramic particles in order to control the consistency desired.

**Table 4**

| Packability Force for Composite Materials | |
|---|---|
| Materials | Packability Force (Newtons) |
| Exp. Doughnut shape (62% wt) | 109.00 ± 17.9 |
| Exp. Mono-sized spherical shape (68% wt) | 4.60 ± 0.26 |
| Surefil | 31.29 ± 1.64 |
| Herculite XR Unidose | 26.70 ± 2.67 |
| Solitaire | 23.26 ± 2.80 |
| Tetric Ceram | 20.36 ± 1.24 |
| TPH Compules | 10.78 ± 1.26 |

The unique-shaped ceramic particle produced in the form of a multi-dimpled zirconium, barium and strontium silicate which provides mechanical locking for the resin matrix, also provides appropriate radiopacity.

To illustrate the production of the ceramic' particles of this invention the following example is given. The filler particles are produced as either ovoid or round discs with a depression or hole through the centre (doughnut-shaped) at least about seventy five percent of the particles produced will have a hole (Figure 1). The external size of these shapes ranges from about 0.2 up to about 20µm, with a mean size of about 5µm and they are chemically composed Of SiO₂. However, a small percentages of other ions such as Na and K may be incorporated (0.5 to 2%) in order to reduce the hardness. Barium, strontium, lanthanide, samarium, dysprosium, or terbium oxides may also be incorporated or coated onto the surface in order to produce a composition which will produce X-ray opacity. The particles are synthesized from a silicate suspension (concentration 10-40 wt% Si02) which may also contain the additional elements if required. The method involves pumping the solution under pressure (of about 70 p.s.i.) through a nozzle (of about 0.5-0.7 mm diam.) at a flow rate of about 10cc per minute into a chamber which is held at a temperature of about 200°C. The small droplets of solution are rapidly heated such that the vapour is eliminated from the external surface of the droplet with a small amount located internally due to the poor thermal conductivity of the silica particle. The final quantity of moisture from the centre of the particle is eliminated causing a hole to be produced in the particle. Final heating (at about 110-120°C) in a second chamber with a partial vacuum of about 10 p.s.i. consolidates the hollow disc shape.

The flow rate for the solution and the nozzle and the chamber temperatures as well as the concentration of the solution or suspension are important to the formation of the desired particle shape and size. A further stage follows in which the particles are heated in a crucible in a furnace for about 24 hours at a temperature of about 600°C. This completes the conversion of the "silica gel" into "silica glass", and also completes in some cases the formation of the holes in the discs. The smooth ovoid or round doughnut ringshaped particles or discs provide a lower residual stress within the matrix resin following polymerization than would be the case with conventional irregular shaped filler particles.

This invention provides a method for producing discrete unique ceramic particles having specific size, shape and chemistry. These particles will be used as the reinforcing phase in a resin-matrix composite for use as a dental filling material. Any resin material suitable for use in the oral cavity is within the scope of the invention. For example, those resins described in US Patent Nos. 4,514,174; 5,338,773 and 5,710,194 are useful.

Similar doughnut shaped particles can also be made from alumina (aqueous suspension of Al₂0₃) and these can be used in resin matrix composites or be incorporated into a glass to produce an alumina/glass composite system of enhanced strength for use as a biomaterial to replace natural hard tissues.

The various unique-shaped ceramic particles can be synthesized for incorporation into various cements of the carboxylate or phosphate type.

Further, the various composite and cement systems mentioned can have special application in a wide range of varied commercial and industrial uses outside the field of dental and medical use.

The surface of the ceramic particles is preferably coated with a silane coupling agent such as 3-(Methacryloxypropyl)-trimethoxysilane. This will be achieved by the particles in a solution of the silane compound and subjecting it to a drying process. The particles may also be treated with a plasma cleaner in order to aid the wetting of the silicate with the organic monomers. Plasma cleaning, involves exposing the surface of the substrate to a gas discharge, which provides a gentle yet thorough scrubbing of the surface removing contaminants and increasing the surface energy may be used.

The sub micron sized spherical filler particles are produced by precipitation from a silica or alumina alkoxide solution by pH control. These smaller particles may be blended together with the unique shaped particles together with conventional glass filler in varying proportions in order to produce a desired packing density, of 75 to 80% by weight of the total filler. The colour and opacity produced with this composite system in the absence of any shading pigments has been found to resemble closely that of natural teeth, and to be close to typical commercial dental composite basic universal shades. The system can thus very easily be modified by incorporation of shading pigments according to the known art to product additional shades which may be required. The special unique-shaped particles may also be blended together (5 to 35 wt%) with finely divided inorganic material such as a barium aluminosilicate glass or other glass having an effective amount of radiopaque oxide that renders the resultant glass radiopaque to X-rays according to the known dental art such as in U.S. Pat. No. 3,911,581.

The unique shaped ceramic particles (5 to 35 wt%) are then blended with conventional glass filler under vacuum, or any other suitable method, with a resin system such as bis phenol dimethacrylate, BIS-GMA, TEGDMA, propyl methacrylate-urethane or diethylene glycol dimethacrylate, or hexamethylene diisocyanate adduct of the diglycidyl methacrylate adduct of bisphenol A, according to, for example, U.S. Pat. No. 3, 629, 187. The photo-curing composite material will use camphoroquinone or similar system in order to produce polymerization.

Tests have been conducted in order to evaluate the elastic moduli and Poisson's ratio for the experimental composite systems of the above type. The purpose of these tests were to determine the influence of type and volume of ceramic fillers on the dynamic moduli of elasticity and Poisson's ratio for the experimental composite materials. Considerations of the fundamental property of modulus of elasticity and fracture toughness for the matrix resin, as well as for blends of the various unique filler with different matrix combinations together with the influence of silanization have been used to optimize the composite systems. Studies of the moduli of elasticity and fracture toughness of experimental composite materials have been able to indicate the effectiveness of the stress/strain transfer from the matrix to the filler particles. Materials with a higher modulus of elasticity and fracture toughness are required for restorations placed in back (molar) teeth. The effectiveness of mechanical locking of ceramic particles into the resin matrix was evaluated using an ultrasonic method. Experimental composite formulations were evaluated in which the ceramic filler types were synthesized by wet chemistry in which some particles were spherical (essentially mono size 0.3-0.4 µm) and others were non-spherical, non-angular, smooth-surface particles of doughnut shape (particle size 1.0-8.0 µm, average particle size of 5.0 µm). The surface areas for both filler types were respectively, 9.66 (m²/g) and 66.58 (m²/g). Tests with no silane treatment of ceramic filler particles were undertaken and compared with silane treated filler in order to allow study of the influence of the size and shape of filler particle in terms of mechanical locking alone. The filler loading was also varied. Significant effects were established for mechanical locking and silane treatment and the contribution of the ceramic filler to the elastic modulus.

The matrix resins to be used with the unique-shaped particles of this invention can be mixtures of BIS-GMA and TEGDMA, or urethane dimethacrylates and large oligomeric structures of BIS-GMA-urethanes may also be used. Combinations of these materials and the like may be employed. The materials of this invention will have a blend of the unique shaped glass, glass-ceramic or ceramic particles combined with conventional glass filler if desired dispersed in a polymerizable synthetic organic resin matrix. The monomer materials being blended together with the inorganic (filler) reinforcing phase such as a mullite, alumina, calcium aluminosilicate, zirconium silicate, barium silicate, titanium silicate or strontium aluminosilicate glass, glass-ceramic or ceramic unique-shaped particles. Preferably blending of large and small particles (distribution, from 0.04 to 10 µm) should be used to obtain optimum packing density and mechanical properties. However, a small proportion of some particles in the range 10 to 50 µm can also be incorporated to improve translucency and other physical properties. Various blends of particle size and shape can be used to achieve maximum loading density. The size and distribution of the filler particles and the refractive index of filler and matrix resin should be optimized to give appropriate translucence for natural aesthetic results. The filler particles of such composites should preferably be surface treated to provide adhesion between the resin matrix and the glass or ceramic filler particles. Adhesion being achieved by using a silane (organo functional adhesion promoter) treatment. The composite systems of this invention may also contain 2-25% (preferably 5-10%) of fumed silica to adjust viscosity and handling characteristics. This sub-micron silica also being treated with a silane coupling agent to reduce the uptake of water by the large surface area. Such composite materials may use photopolymerizing systems activated by visible light in which a light sensitive absorber such as camphorquinone is used together with an aliphatic amine accelerator. However, chemically activated composite systems using the N,N-dimethyl-para-toluidine and benzoyl peroxide or similar system for chemical activation may also be appropriate, as will the heat curing systems since the composite systems using the unique filler may also have application in the from of indirect dental restorative devices such as inlay or onlays fabricated outside the mouth. Unless otherwise stated, all "percents", % and the like are weight percents.

## Claims

1. A filled dental material having improved mechanical properties comprising:
a resin matrix and a filler component;
wherein said filler component comprises from 5 to 35 percent by weight of ceramic particles having a preselected shape obtainable by a wet chemical synthesis;
said preselected shape selected from the group consisting of spheres, doughnuts, multi-dimples, hollow spheres, nuggets, rods, fibers, and mixtures thereof;
wherein the filled dental material is improved due to mechanical interlocking of the ceramic particles within the matrix and wherein doughnuts, multi-dimples, porous hollow spheres, or nuggets are present.

2. A filled dental material as in claim 1, wherein said multi-dimpled shaped particles are porous.

3. A filled dental material as in claim 1, wherein said multi-dimpled shaped particles are solid.

4. A filled dental material as in claim 1, wherein said filler particles are produced by wet chemical synthesis of a material selected from the group consisting of zirconium, silica, barium, titanium, strontium, alumina, mullite or mixtures thereof.

5. A filled dental material as in claim 1, wherein said filler particles are a mixture of silica and from about 3 to about 40 percent by weight of ZrO₂.

6. A filled dental material as in claim 4, wherein said filler particles are a mixture of silica and from about 10 to about 80 percent by weight of BaO.

7. A filled dental material as in claim 4, wherein said filler is a mixture of silica and from about 3 to about 40 percent by weight of TiO₂.

8. A filled dental material as in claim 4, wherein said filler is a mixture of silica and from about 10 to about 80 percent by weight of SrO.

## Patentansprüche

1. Gefülltes Dentalmaterial mit verbesserten mechanischen Eigenschaften, umfassend: eine Harzmatrix und eine Füllstoffkomponente;
worin die Füllstoffkomponente 5 bis 35 Gew.-% Keramikteilchen mit einer bestimmten Form enthält, erhältlich durch ein nasschemisches Syntheseverfahren;
wobei die bestimmte Form ausgewählt ist aus der Gruppe bestehend aus Kugeln, Ringwulsten, Multidellen, Hohlkugeln, Nuggets, Stäbchen, Fasern und Mischungen davon;
wobei das gefüllte Dentalmaterial aufgrund eines mechanischen in einander Greifens der Keramikteilchen innerhalb der Matrix verbessert ist, und worin Ringwulste, Multidellen, poröse Hohlkugeln oder Nuggets vorhanden sind.

2. Gefülltes Dentalmaterial nach Anspruch 1, worin die Multidellen-förmigen Teilchen porös sind.

3. Gefülltes Dentalmaterial nach Anspruch 1, worin die Multidellen-förmigen Teilchen dicht sind.

4. Gefülltes Dentalmaterial nach Anspruch 1, worin die Füllstoffteilchen nach einem nasschemischen Syntheseverfahren aus einem Material gebildet werden, das ausgewählt ist aus der Gruppe bestehend aus Zirconium, Siliciumdioxid, Barium, Titan, Strontium, Aluminiumoxid, Mullit oder Mischungen davon.

5. Gefülltes Dentalmaterial nach Anspruch 1, worin die Füllstoffteilchen eine Mischung aus Siliciumdioxid und etwa 3 bis etwa 40 Gew.-% ZrO₂ sind.

6. Gefülltes Dentalmaterial nach Anspruch 4, worin die Füllstoffteilchen eine Mischung aus Siliciumdioxid und etwa 10 bis etwa 80 Gew.-% BaO sind.

7. Gefülltes Dentalmaterial nach Anspruch 4, worin die Füllstoffteilchen eine Mischung aus Siliciumdioxid und etwa 3 bis etwa 40 Gew.-% TiO₂ sind.

8. Gefülltes Dentalmaterial nach Anspruch 4, worin die Füllstoffteilchen eine Mischung aus Siliciumdioxid und etwa 10 bis etwa 80 Gew.-% SrO sind.

## Revendications

1. Matériau dentaire chargé ayant des propriétés mécaniques améliorées, comprenant :
une matrice résineuse et un composant de charge ;
dans lequel ledit composant de charge comprend 5 à 35 pour cent en poids de particules de céramique ayant une forme prédéterminée, pouvant être obtenues par une synthèse chimique par voie humide ;
ladite forme prédéterminée étant choisie dans le groupe constitué de sphères, tores, formes multialvéolées, sphères creuses, pépites, tiges, fibres et leurs mélanges ;
dans lequel le matériau dentaire chargé est amélioré en raison du blocage mécanique des particules céramiques à l'intérieur de la matrice, et dans lequel des tores, particules multialvéolées, sphères creuses poreuses ou pépites sont présents.

2. Matériau dentaire chargé selon la revendication 1, dans lequel lesdites particules de forme multialvéolée sont poreuses.

3. Matériau dentaire chargé selon la revendication 1, dans lequel lesdites particules de forme multialvéolée sont pleines.

4. Matériau dentaire chargé selon la revendication 1, dans lequel lesdites particules de charge sont produites par synthèse chimique par voie humide d'une matière choisi dans le groupe formé par le zirconium, la silice, le baryum, le titane, le strontium, l'alumine, la mullite ou leurs mélanges.

5. Matériau dentaire chargé selon la revendication 1, dans lequel lesdites particules de charge sont un mélange de silice et d'environ 3 à environ 40 pour cent en poids de ZrO₂.

6. Matériau dentaire chargé selon la revendication 4, dans laquelle lesdites particules de charge sont un mélange de silice et d'environ 10 à environ 80 pour cent en poids de BaO.

7. Matériau dentaire chargé selon la revendication 4, dans laquelle ladite charge est un mélange de silice et d'environ 3 à environ 40 pour cent en poids de TiO₂.

8. Matériau dentaire chargé selon la revendication 4, dans laquelle ladite charge est un mélange de silice et d'environ 10 à environ 80 pour cent en poids de SrO.
